(19) Europäisches Patentamt
European Patent Office
Office européen des brevets

(11) Publication number: 0 109 428
B1

(12) # EUROPEAN PATENT SPECIFICATION

(45) Date of publication of patent specification: **06.04.88**

(51) Int. Cl.$^4$: **C 12 N 15/00**, C 12 N 1/00, C 12 P 21/00, C 07 H 21/04, A 61 K 45/02, G 01 N 33/531

(21) Application number: **83901883.5**

(22) Date of filing: **24.05.83**

(86) International application number: **PCT/US83/00797**

(87) International publication number: **WO 83/04262 08.12.83 Gazette 83/28**

(54) **Cloning vector with indicator genes which have been inactivated by frameshift.**

(30) Priority: **25.05.82 US 382043**

(43) Date of publication of application: **30.05.84 Bulletin 84/22**

(45) Publication of the grant of the patent: **06.04.88 Bulletin 88/14**

(84) Designated Contracting States: **AT BE CH DE FR GB LI LU NL SE**

(56) References cited:
**EP-A-0 011 560**
**GB-A-2 071 671**

**Cell, vol. 20, June 1980 L. Guarente et al.: "Improved methods for maximizing expression of a cloned gene: a bacterium that synthesizes rabbit-beta-globin", pages 543-553, see pages 549-552**

**Science, vol. 209, 19 September 1980 L. Guarente et al.: "A technique for expressing eukaryotic genes in bacteria", pages 1428-1430, see pages 1428 and 1429**

(73) Proprietor: **BRANDEIS UNUVERSITY
415 South Street
Waltham, MA 02154 (US)**

(72) Inventor: **ROSBASH, Michael M.
665 Grove Street
Newton, MA 02162 (US)**

(74) Representative: **Holdcroft, James Gerald, Dr. et al
Graham Watt & Co. Riverhead
Sevenoaks Kent TN13 2BN (GB)**

(56) References cited:
**Gene, vol. 19, 1982 J. Vieira et al.: "The puc plasmids, an M13mp7-derived system for insertion mutagenesis and sequencing with synthetic universal primers", pages 259-268, see the entire document**

**EP 0 109 428 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field

This invention is in the fields of biology, medicine, and genetic chemistry.

Background Art

Proteins are created by a complex process that may be roughly divided into three stages. Those stages are commonly designated as transcription, processing, and translation, and they are summarized very briefly below.

The process of transcription involves the creation of a strand of RNA from a DNA template [1]. The DNA double helix separates temporarily into two strands, and an enzyme called "RNA polymerase" travels along one strand of DNA in the 5′ to 3′ direction. As it moves, the polymerase identifies each exposed base on the DNA strand. The four bases are adenosine, thymine, cytosine, and guanine (designated as A, T, C and G, respectively). The polymerase creates a strand of RNA with corresponding bases as follows:

| DNA Template | Transcribed in mRNA |
|---|---|
| A | U (uracil |
| T | A |
| C | G |
| G | C |

The length of the RNA strand created by the polymerase is controlled by transcriptional initiation and termination sites and by other factors which are not entirely understood.

The strand of RNA thus created is called the "primary transcript." In eukaryotic cells, it is processed or "edited" before it leaves the nucleus [2]. This is accomplished by a complex series of steps, some of which are not completely understood. One step of this process involved RNA splicing. Briefly, certain segments of the RNA strand are removed from the strand; the remaining segments are reconnected into a shorter strand which is then suitable for translation into protein. An "intron" refers to a portion of a gene which is transcribed into RNA which is deleted during processing; an intron is not translated into protein. An "exon" refers to a portion of a gene which is conserved throughout the RNA processing stage. The exon-complementary segments are reconnected to each other after the intron-complementary segments have been deleted to form mRNA. The edited mRNA leaves the nucleus and is translated into protein.

The process of translation [3] involves the sequential connection of amino acids by means of peptide bonds, as shown in the following example reaction:

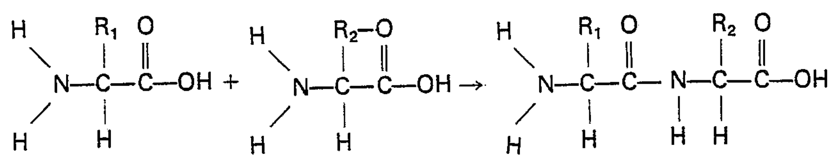

where $R_1$ and $R_2$ represent organic moieties. Only 20 different moieties are used to create the proteins in all living matter.

Every protein or other polypeptide has an end which may be called an "amino terminus," an "N-terminus" or a "left-hand end." The other end of the polypeptide chain may be called the "carboxyl terminus," the "C-terminus," or the "right-hand end."

As used herein the N-terminus of a polypeptide refers to either of the following:

a. a methionine or formyl methionine molecule which is coded for by an AUG start codon, or

b. an amino group or derivative thereof which is at the end of a polypeptide.

If a protein is divided into two portions, they may be regarded as the "N-terminal portion" (which contains the N-terminus) and the "C-terminal portion."

Every indicator polypeptide (as defined below) contains one or more amino acids or amino acid sequences which are essential for the proper functioning of the polypeptide. Such amino acids are referred to herein as an "indicator sequence." As used herein, the "N-terminal portion" of a polypeptide refers to one or more amino acids which are located between the N-terminus and an indicator sequence of the polypeptide. Similarly, the "N-terminal portion" of a gene refers to a polynucleotide sequence which codes for an N-terminal portion of a polypeptide.

The sequence of amino acids in a protein is very important to the proper functioning of the protein. The amino acid sequence is based upon the genetic code, which is identical for all types of cells. The bases in a strand of mRNA are divided into groups of three; each group of three is called a "codon." Using the four

bases of RNA, there are 64 possible codon sequences. Each sequence corresponds to a single type of amino acid. The entire genetic code has been identified [4]; for example, the sequence UCA (specified in the 5′ to 3′ direction) codes for the amino acid serine. More than one codon sequence may code for a single amino acid; for example, four different codons (CCU, CCC, CCA, and CCG) code for proline.

One codon, AUG, is commonly called the "start codon" or a "translational initiation site." When a strand of mRNA passes through a ribosome, the process of peptide bonding does not commence until an AUG sequence is recognized. At that point, a methionine molecule (which is an amino acid) becomes the N-terminus of the protein that is being created. The next three nucleotides of the mRNA are read together as the next codon, and the corresponding amino acid is connected by a peptide bond to the methionine. The AUG start codon determines the "reading frame" or "register" of the mRNA; in other words, the AUG start sequence determines how the nucleotides will be categorized into groups of three, each group of three serving as a codon.

Three of the 64 codon sequences are termination signals, commonly called "stop" codons. Those three codon sequences are UAA, UAG, and UGA. When a ribosome is translating a strand of mRNA, it continues adding amino acid molecules to the protein until it reaches a stop codon. It then releases the protein, and the amino acid which was coded for by the preceding codon becomes the C-terminus of the protein.

A segment of DNA which does not contain any stop codons is referred to herein as an "open reading frame." Stop codons do not occur within the coding regions of exon segments in the proper reading frame; otherwise, stop codons would cause a premature truncation of the protein being translated, which might impair or destroy the functioning of the protein. The only exonic stop codon in the proper reading frame is at the terminal 3′ sequence of the exon.

Stop codons may be created by various types of mutation. For example, if a gene suffers a deletion of a nucleotide, the translation of the sequence can be totally altered, as indicated in the following example deletion:

| | |
|---|---|
| Normal protein | Asp His VAl Ala |
| Normal RNA | GAU/CAU/GUA/GCA/ |
| Altered RNA | GAU/AUG/UAG/CA |
| Altered protein | Asp Met term |

Stop codons are presumed to occur randomly within non-coding DNA. They are also presumed to be created randomly when a gene is mutated or translated in the wrong frame.

Beta-galactosidase

In general, enzymes are biochemically active proteins which catalyze specific biochemical functions. For example, the enzyme β-galactosidase (B—G) cleaves lactose (which is a disaccharide, i.e., a sugar molecule with two carbohydrate rings) into two monosaccharide rings, galactose and glucose, as shown in the following formula:

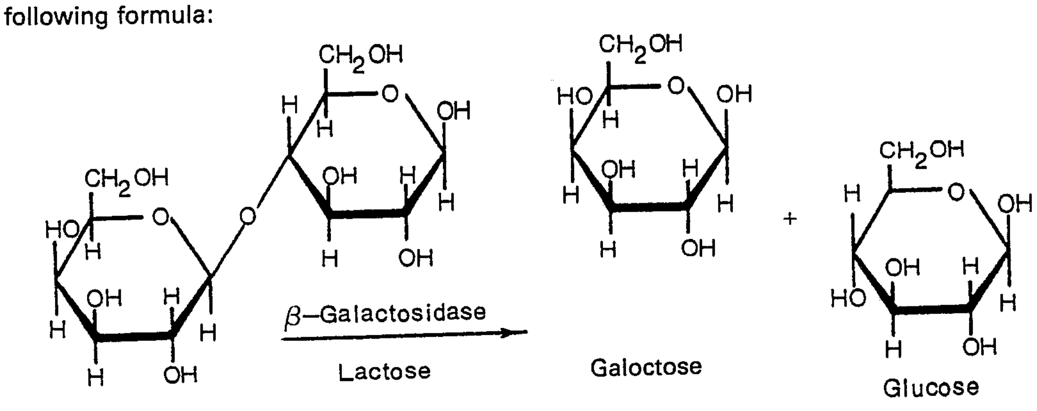

Lactose

Galoctose

+

Glucose

The enzyme B—G occurs in the commonly used bacteria *Escherichia coli*. It is coded for by the B—G gene, commonly called the lac-z gene.

Mutant strains of *E. coli* are commonly used which are lac-$z^-$, i.e., the β-G gene is not functional. Such cells can be grown easily using nutrients, such as glucose, which does not require metabolism by the β-G enzyme.

It is simple and convenient to distinguish lac-$z^+$ cells from lac-$z^-$ cells, by growing the cells on indicator plates which are commercially available, such as "lactose MacConkey" indicator plates. Both $z^+$ and $z^-$ cells can grow on such plates. Colonies of cells that have high levels of β-G activity (more than about 1000 units/cell) turn red. Colonies that have low levels of β-G activity are colorless.

Indicator plates may also contain one or more selective agents, such as an antibiotic such as ampicillin. For example, an indicator plate which contains ampicillin allows for the growth of *E. coli* cells which contain the enzyme β-lactamase. This allows a convenient method of selecting cells which contain plasmids that code for β-lactamase.

The β-G enzyme often remains functional even if the N-terminus is removed and replaced by another polypeptide. It has been shown that an N-fragment which contains up to about 40 amino acids can be replaced by a wide variety of other polypeptides, without seriously affecting the β-G activity of the remaining C-fragment of β-G enzyme [5]. This allows for the creation of a fusion protein with β-G activity, by means of creating a hybrid or "chimeric" gene using recombinant DNA techniques or other genetic methods.

Gene Modification

Genetic recombination has been studied intensively in recent years. Various methods have been described in numerous articles [6] and several patents [7]. Several techniques of particular interest to this invention are described below; however, these are not the only techniques which can be utilized in the method of this invention. Other techniques which are known to those skilled in the art, or which are hereafter discovered, may be used to perform the methods of this invention.

A double-helical strand of DNA can be cut ("cleaved" or "opened") by an enzyme called an "endonuclease" (i.e., an enzyme which is capable of breaking the phosphodiester bonds in both strands of a DNA double heli). Such nucleases often recognize a specific sequence of base pairs. For example, two such nucleases cut DNA in the following manner:

C C C | G G G
G G G | C C C
Sma-1

G | G A T C C
C C T A G | G
Bam-H1

The nuclease designated "Sma-1" creates a "flush" end; the nuclease designated "Bam-H 1" creates a "sticky" or "cohesive" end. A wide variety of endonucleases are known to exist, most of which have been correlated with a sequence of base pairs (usually from four to six base pairs in a specific sequence) that serve as cleavage sites.

If desired, it is possible to convert a sticky end into a flush end in at least two different ways. One way is to add nucleotides to the cleaved DNA with DNA polymerase, which is able to supply the "missing" bases, as shown in the following example:

C G — DNA polymerase → G G G A T C
C C C T A G — C C C T A G

Another method is to contact the cleaved DNA with an "adapter", which is a sequence of nucleotides in a prearranged order. For example, if the adapter sequence GATCCCCGGG is mixed in solution, it will anneal to itself to form the following double-helical fragment:

G A T C C C C G G G
G G G C C C C T A G

The fragment will litigate with the sticky ends created by the nuclease "Bam-H1" to create the following sequence:

G G | G A T C C C C G G G | G A T C C
C C C T A G | G G G C C C C T A G | G
Bam-H1 Sma-1 Bam-H1

This sequence contains a Sma-1 cleavage site which is surrounded or "bracketed" by Bam-H1 cleavage sites in both directions.

Several plasmids with modified β-G genes have been created. For example, L. Guarente et al. have reported [6] and [8] three plasmids (pLG200, pLG300, and pLG400) with carboxyl terminal regions of lac z genes fused to portions of lac i genes, with a Hind III cleavage side near the 5′ end of the lac i gene.

The present invention provides a DNA vector consisting essentially of, in 5′ to 3′ orientation: a) a bacterial promoter, ribosomal binding site and translational start signal; b) a structural gene coding for an indicator polypeptide, or a portion of the structural gene containing at minimum the region of the gene which codes for a fragment of the indicator polypeptide which is essential for its functional activity; and c) an oligonucleotide insert, located between the translation start signal and the region of the structural gene which does for the fragment of the indicator polypeptide which is essential for its functional activity, which insert causes an out of phase shift in the reading frame of the structural gene or portion thereof and contains a recognition site for a restrictive endonuclease. The recognition site of the oligonucleotide insert is for example not found elsewhere in the vector.

It will be realised that a DNA vector according to the invention contains a start codon which is situated downstream of the promoter and ribosomal binding site and upstream of the frameshift insert and of the gene for the indicator polypeptide. The vectors described in Guarente et al. [6] and [8] do not contain a start codon upstream of the lac z gene. In these vectors the start codon must be supplied by the inserted DNA.

In Guarente et al. a two-step procedure is required: first in order to place the eukaryotic sequence adjacent to the lac z gene and second in order to insert a promoter and ribosomal binding site upstream of the eukaryotic sequence. In the prior method, a specific junction must be made between the eukaryotic coding sequence and the lac z gene.

Likewise, an appropriate initiating methionine codon must be available (see Figure 2 of reference [6(b)]). Thus a methionine initiating codon must be available in the inserted eukaryotic piece and DNA sequence information is required in order to make the appropriate two-step construction. Placing the initiating methionine codon in the vector obviates the requirement that the eukaryotic coding piece contain its own initiating methionine. Most importantly, combining this vector itself unable to produce a β-galactosidase because the initiating methionine is not in frame with the β-galactosidase, with shotgun cloning techniques eliminates any requirement for information about the coding DNA to be cloned. Moreover one does not even need to know which piece or pieces contain coding as opposed to non-coding DNA.

Although the basic shotgun isolation technique is known and also that DNA vectors containing β-galactosidase indicator gene will respond to the insertion of heterologous DNA from a library of DNA fragments, our procedure is the only one to provide for insertional *activation* which requires not only the insertion of a piece of eukaryotic DNA but the insertion of a piece of open reading frame DNA.

This procedure of insertional activation not only distinguishes clones carrying coding DNA from those carrying non-coding DNA (or those containing no insert), but also provides for expression of the open reading frame fragment at the same moment. Thus, this one-step shotgun cloning procedure takes the two-steps shown in the prior references and compresses them into one step; it eliminates the requirement for an initiating methionine in the eukaryotic portion, it eliminates the nibbling step in the prior art to place that initiating methionine at the correct distance from the ribosomal binding sequences provided by the fragment in step B of Figure 1 of reference [6(b)], and most importantly, it provides a totally novel, step of insertional activation which requires an open reading frame piece of DNA. This latter advantage obviates in one clean "shotgun step" all of the information required by the prior references in order to make the construct as shown in Figure 2 of reference [6(b)]. Again, the prior cloning procedure cannot be accomplished in the absence of detailed sequence information about the piece of eukaryotic DNA to be cloned.

The invention also comprehends method aspects.

There now follows a description of embodiments of the invention. This description is given by way of example only and not by way of limitation of the invention.

Disclosure of the Invention

This invention relates to a cloning vector with an inactivated gene. In its unmodified "wild-type," the gene codes for an indicator polypeptide, such as β-galactosidase (β-G). The gene in the cloning vector has a cleavage site which allows for the insertion of a DNA fragment into the gene. The gene has also been inactivated by an addition or deletion which causes a frameshift.

If a DNA fragment with the following characteristics:

a. an open reading frame, i.e., the absence of stop codons, and

b. the proper number of nucleotides to cause a correcting frameshift

is inserted into the cleavage site, the gene may be activated. The first condition virtually ensures that the only types of lengthy DNA fragments that can activate the gene are exon segments (i.e., DNA segments that are transcribed into mRNA which is translated into protein) that are being read in the proper register. The second condition is presumed to be satisfied randomly with a probability of 1/3.

A large number of polynucleotide fragments may be created by fragmenting the genetic material of an organism of interest, such as a virus. The fragmentation may be performed using known techniques, such as sonication or shearing. If desired, the resulting fragments may be processed by electrophoresis, centrifugation, etc. Using techniques known to those skilled in the art, the DNA fragments may be inserted

into numerous cloning vectors at the desired location in the inactivated gene. The resulting plasmids may be inserted into suitable cells, using known techniques.

The cells are cultured in a manner which allows for the identification of cells which contain activated genes, i.e., genes which code for one or more indicator polypeptides. For example, if the indicator polypeptide is β-G, the cells may be grown on indicator plates which cause lac $z^+$ colonies to exhibit a red color, while $z^-$ colonies are white. The lac $z^+$ cells are very likely to contain plasmids which contain inserted DNA segments that are of great interest.

The cells themselves are of great interest. They may be utilized for several purposes, including:

a. creating proteins (such as vaccines and other antigens) that are coded for by the organism of interest;

b. creating antibodies which bind to said proteins and to the organism of interest;

c. identifying the amino acid sequence of such proteins;

d. identifying the nucleotide sequence and reading frame of the DNA of the organism of interest.

Brief Description of the Drawings

Figure 1 illustrates the creation of pMR2, an intermediate cloning vector.

Figure 2 illustrates the creation of pMR100, cloning vector of this invention.

Best Mode of Carrying Out the Invention

One preferred embodiment of this invention utilizes a cloning vector designated as pMR100. This vector contains a lac operon with the following characteristics:

1. functional sites for promotion, translation initiation, ribosome binding, and translation termination;

2. a TAC nucleotide sequence which is transcribed into an AUG start codon;

3. a C-terminal region of the β-G gene which has been inactivated by a frameshift caused by the insertion of a nucleotide segment (comprising ten nucleotides) into the N-terminal regin of the β-G gene; and

4. a nucleotide sequence within the N-terminal region of the β-G gene which allows for a single Sma-1 cleavage site bracketed by two Bam-Hi cleavage sites, as shown below:

```
G G |G A T C C C C G G G |G A T C C
C C C T A G| G G G C C C C T A G| G
```

Cloning vector pMR100 also contains the following characteristics:

1. a gene which codes for β-lactamase, thereby providing resistance to certain antibiotics such as ampicillin;

2. the absence of Sma-1 cleavage sites other than the site within the inactivated β-G gene.

DNA fragments were created by cleaving a plasmid, pBR322, with a restriction endonuclease, HaeIII. Each plasmid was cleaved into 22 fragments, ranging in length from 7 nucleotides to 587 nucleotides. These fragments were contacted with pMR100 cloning vectors which had been cleaved by Sma-1, and with DNA ligase to cause the cleaved phosphodiester bonds to be reconnected. This created plasmids with inserted DNA, as well as religated vectors with no inserted DNA. The religated vectors with no inserted DNA remained lac $z^-$.

The mixture of plasmids and religated vectors were then contacted with *E. coli* cells that are devoid of lac $z^-$ gene DNA. The cells were treated with calcium chloride to promote the uptake of plasmids and religated vectors. The transformed cells were then cultured to promote the replication of plasmids within the cells, causing numerous copies to be present in most cells.

The resulting cells were then seeded onto lactose MacConkey indicator plates, at relatively low densities. A small fraction of the seeded cells grew into colonies that were red, indicating that they contained β-G activity.

Based upon the nucleotide sequence of the pBR322 plasmid, it was predicted that only 3 of the 22 HaeIII fragments were capable of activating the β-G gene in the pMR100 cloning vectors. Those three fragments all have (3×-1) nucleotides, where x is an integer; the actual fragment sizes were 80, 89, and 104 bases. In addition, none of the three fragments contains a stop codon or creates a stop codon when inserted into a Sma-1 cleavage site.

The colonies were analyzed to compare the experimental results with the predicted results. The results agreed perfectly; all three predicted fragments were found in red colonies, and no DNA fragments other than the three predicted fragments were found in red colonies.

The cloning vector and the methods of this invention can be utilized to create a wide variety of very useful products, including:

1. genetic material, such as plasmids and segments of DNA. Such genetic material can be removed from the cells that were used to identify it, and processed in a variety of ways. For example, the entire plasmid may be useful for insertion into other types of cells. Alternately, the exogenous DNA segment may be removed from the plasmid, for example, by use of a restriction endnuclease which cleaves the plasmid at two sites that bracket the insertion cleavage site. Either the plasmid or the extracted DNA segment may be treated by genetic recombination techniques that are currently known or hereafter discovered.

2. cells which contain DNA segments of interest. Of course, only a few cells are likely to be directly identified by the methods of this invention. However, such cells can be cultured into billions of cells, each of which will contain the same valuable genetic material, with very limited exceptions. Any cell which is a descendent of, or is otherwise derived from, a cell which is identified by the method of this invention, is within the scope of this invention.

3. polypeptides, such as antigenic proteins which can serve a variety of purposes, such as vaccination agents or agents for the production of polyclonal or monoclonal antibodies. Such polypeptides may be expresed by, or be capable of being removed from, the cells identified by the methods of this invention, or by any cells which descend from or are otherwise derived from cells identified by this invention.

4. antibodies which are capable of binding to the polypeptides that can be created by this invention.

5. assay kits utilizing the polypeptides or antibodies mentioned above.

As used herein, the term "indicator polypeptide" includes any protein, protein fragment, or other polypeptide which has activity or other characteristics that allow cells which produce such polypeptides to be distinguished from (1) cells which do not produce such polypeptides, or (2) cells which produce polypeptides which for any reason do not have the same characteristics as the indicator polypeptides. For example, an indicator polypeptide may comprise an enzyme which causes a color change or other chemical reaction when contacted with selected chemicals, or an enzyme which increases the survivability of a cell in the presence of a selective agent, or an amino acid sequence which modifies the rate of transport, expression, or degradation of the polypeptide.

As used herein, the term "fragment" refers to a polynucleotide or polypeptide sequence that has been dissociated from the surrounding DNA or protein, respectively. By comparison, the term "segment" refers to any polynucleotide or polypeptide sequence, regardless of whether it has been dissociated from the surrounding DNA or protein.

As used herein, DNA includes complementary DNA (cDNA) which has been prepared from a template of RNA or single stranded DNA.

As used herein, a "cloning vector" is a DNA molecule which contains, inter alia, genetic information which insures its own replication when transferred into a cell. Examples of transfer vectors commonly used are the DNA of bacteriophages, and plasmids (i.e., non-chromosonal loops of DNA that exist inside cells). For convenience, the term "plasmid" is used hereafter in a slightly different sense; it includes any cloning vector that contains a segment of inserted DNA.

An essential step in several of the claims comprises creating nucleotide fragments. There are several techniques known to those skilled in the art for performing this step. Such techniques include:

1. the fragmentation of DNA by means such as sonication, shearing, or contact with endonucleases or substances which cause bonds between nucleotides to break:

2. the creation of double-stranded DNA from a template comprising RNA or single-stranded DNA, by means such as contact with substances such as reverse transcriptase or DNA polymerase(s). Such steps might be performed either before or after the RNA or ssDNA is fragmented.

3. the creation of bonds between nucleotides, oligonucleotides, or polynucleotides, or their analogs, by enzymatic or non-enzymatic means, with or without the use of templates.

## Example 1

Construction of pMR100 Cloning Vector

The plasmid pLG 400 (obtained from L. Guarente, Harvard Univ., described by L. Guarente et al, *Cell 20*:543, 1980) was digested with endonucleases Sma-I and Hind-III (Bethesda Research Laboratories (BRL), Bethesda, MD). The largest of the two resulting DNA fragments (approximately 8.8 kb long) was isolated by agarose gel electrophoresis, electroeluted in 0.05 × buffer comprising 90 mM Tris, 90 mM boric acid, and 4 mM EDTA. The eluted DNA was bound to DEAE-sepharose, and eluted with 1M NaCl, 10 mM Tris, 1 mM EDTA buffer. The fragment was precipitated in 95% ethanol, washed in 70% ethanol, and dried by lyophilization.

The plasmid pKB252 (obtained from L. Guarente, described by K. Backman et al, *PNAS 73:* 4174, 1976) was digested by endonucleases HAE—III and Hind-III (obtained from BRL). The largest fragment (about 1.1 kb) was purified in the same manner as above.

The two purified fragments were ligated together using $T_4$ ligase (from BRL) overnight at 16°C, as diagrammed in Figure 1.

The ligated mixture was used to transform a strain of *E. coli* bacteria, designated as LG90. This bacterium is sensitive to ampicillin, and it does not contain a lac operon. The transformation was promoted by a standard technique using 50 mM calcium chloride.

A clonal colony that was lac-$z^+$ (red color) on MacConkey indicator plates (Difco, Detroit, MI) was selected and cultured. Plasmids were removed from the cells by lysing the cells with detergent, followed by several purification steps. They were tested by various endonucleases; the results confirmed the structure indicated in Figure 1. These plasmids were designated as pMR2.

The plasmid pMR2 was linearized by Bam-HI (New England Biolabs, Beverly, MA), extracted in phenol chloroform, precipitated in 95% ethanol, washed in 70% ethanol, and lyophilized. The resulting linear DNA was incubated for 3 hours at 22°C in the presence of $T_4$ ligase and a 50 fold molar excess of a synthetic oligo nucleotide with the following sequence: GATCCCCGGG. This results in a Sma-I cleavage site bracketed by two proximate Bam-HI cleavage sites. This insertion causes a frameshift (3 x + 1) which inactivates the C-terminal portion of the lac-z gene. The resulting plasmid was designated as cloning vector pMR100. It was used to transform LG90 cells, which tested lac-$z^-$ on MacConkey indicator plates.

Example 2:

Preparation of DNA Fragments

The plasmid ptkSVB was obtained from D. Livingston, Harvard University. The plasmid was digested with endonuclease Hind-III (from BRL). The smaller of the two resultant fragments was gel purified as described in Example 1. A 1169bp segment was fragmented by sonication using a Heat Systems-Ultrasonics sonifier, setting 6, for 180 seconds, in 5 ml of 0.2 M NaCl, 10 mM Tris, 1 mM EDTA. The sonicated DNA was concentrated to 0.4 ml using a DEAE-sepharose column as described in Example 1. This DNA was precipitated and washed in ethanol and lyophilized. The DNA was then treated with the nuclease BAL—31 (obtained from BRL) using .01 units/mg DNA for 5 minutes at 30°C in a 10 µl vol/mg DNA. The DNA was then phenol extracted and ethanol precipitated, resuspended in 10 mM Tris, 1 mM EDTA, and size-fractionated on 10% acrylamide-TBE gels. Fragments of the desired size class (approximately 350—500 bp) were selected using adjacent DNA fragment markers on the same gel electroblotted to DEAE-cellulose paper in 20 mM Tris, 1 mM EDTA for 2 hours at 100 mA. The size-fractionated DNA was eluted using 1 N NaCl-TE, precipitated and washed in ethanol, dried, and resuspended in TE.

Example 3

Insertion of DNA Fragments Into Cloning Vectors

The plasmid pMR100 was linearized by the endonucleases Sma-I (from BRL), digesting for 2 hours at 37°C. The 5′ terminal phosphate groups were removed from the Sma-I ends to prevent religation, using calf intestinal alkaline phosphatase (laboratory stock), digesting at 37°C for 30 minutes. The phosphatased, linearized vector was phenol extracted, ethanol precipitated, and resuspended in 10 mM Tris, 1 mM EDTA at a final concentration of 0.5 mg/µl. The vector was incubated with ptkSVB fragments, prepared as described above, in a ratio of 500 ng vector to 200 ng fragments, in a 10 ml volume using 1 unit T4 ligase overnight at 16°C.

Example 4

Transformation of Recipient Cells

A 2 µl aliquot of the ligation mixture described in Example 3 was put into an eppendorf tube, resuspended in 200 µl of 10 mM Tris, 1 mM EDTA, 0.2 M NaCl, and phenol extricated once. The aqueous layer containing the plasmids was mixed with 10 µg of yeast-tRNA carrier (from Gibco, NY), precipitated and washed in ethanol, dried and resuspended in 5 µl of 10 mM Tris, 10 mM $MgCl_2$, 10 mM $CaCl_2$. This mixture was used to transform 0.05 ml L690 using the 100 mM $CaCl_2$ transformation method of Dagert and Ehrlich (*Gene 6:*23, 1979). The transformants were plated on MacConkey agar plates containing 25 µg/ml ampicillin (Sigma). Colonies of lac-$z^+$ transformants were selected for further characterization after 24 hours incubation at 37°C.

Example 5

Characterization of Plasmid DNA From lac-$z^+$ Clones

Lac-$z^+$ cones from Example 4 were subcultured in 1 ml L broth containing 50 µg/ml ampicillin. Plasmid DNA was extracted from the cells using the method described in *Nucleic Acids Research 5:*298, 1981. The DNA precipitate was resuspended in 25 µl of 10 mM Tris, 1 mM EDTA. Five µl of the DNA was digested with 2 units of BamHI in a 20 µl reaction volume, for 60 minutes at 37°C. The digested DNA was treated with 1 µg/µl RNAase (laboratory stock) for 5 minutes at 37°C, and analyzed on 10% acrylamide TBE gels, run at 300 V for about 90 minutes. The gels were stained in ethidium bromide for 20 minutes and photographed. The size of the DNA insert was compared to size standards run on the same gel. With one possible exception (which may be due to a previously undetected cleavage site) the results of over 50 experiments have correlated well with predicted results.

Example 6

Characterization of the Fusion Proteins

Selected lac-$z^+$ clones were subcultured into 3 ml L broth with 50 µg/ml ampicillin, and incubated at 37°C overnight. The cells were then centrifuged at 10,000 g for 5 minutes; the supernatant was discarded. 150 µl of 1.2 × Laemmli sample buffer was added to each pellet. Each pellet was resuspended by mixing and heated to 98°C for 3 minutes. The protein samples were then drawn into a 22 gauge needle 5 or 6 times to reduce sample viscosity. The samples were tested by electrophoresis on 7.5% acrylamide SDS Laemmli gels, running at 200 V for approximately 3 hours. After electrophoresis, the gel was stained in 0.1% Coomassie blue for 60 minutes, and destained in 10% acetic acid-50% methonol for about 3 hours. The gel was then allowed to swell in 10% acetic acid and then dried using vacuum and heat. The size of the fusion protein was compared to the protein produced in cells containing pMR100 with no inserted DNA.

*References*

1. See, e.g., A. L. Lehminger, *Biochemistry*, 2nd Edition, p. 89 et seq. (Worth Publishers, New York City, 1975); L. Stryer, *Biochemistry*, 2nd Edition, p. 597 et seq. (W. H. Freeman & Co., San Francisco, 1981).
2. See, e.g., Stryer, supra note 1, at p. 702 35 seq.
3. See, e.g., Lehminger, supra note 1, at p. 929 et seq.: Stryer, supra note 1, at p. 619 et seq.
4. See Lehminger, supra note 1, at p. 962; Stryer, supra note 1, at p. 629; U.S. Patent 4,322,499 (Baxter et al., 1982), column 3.
5. E. Brickman et al., *J. Bacteriol. 139*: 13—18 (1979).
6. See, e.g., L. Guarente et al., (a) *Cell 20*: 543—553 (1980) and (b) *Science 209:* 1428—1430 (19th September 1980).
7. See, e.g., U.S. Patent 4,237,224 (Cohen et al., 1980); U.S. Patent 4,322,499 (Baxter et al., 1982).
8. L. Guarente et al., supra note 6, at p. 549 et seq.
9. See, eg., H. L. Bachrach et al., *J. Immunol. 115:* No. 6, p. 1636—1641 (1975); R. H. Meloen, *J. Gen. virol. 45:* 761—763 (1979); M. Breindl, *Virology 46:* 962—964 (1971).
10. See *Science 213:* 623—628 (7 August 1981).

**Claims for the Contracting States: BE CH DE FR GB LI LU NL SE**

1. A DNA vector consisting essentially of, in 5′ to 3′ orientation:

a) a bacterial promoter, ribosomal binding site and translational start signal;

b) a structural gene coding for an indicator polypeptide, or a portion of the structural gene containing at minimum the region of the gene which codes for a fragment of the indicator polypeptide which is essential for its functional activity; and

c) an oligonucleotide insert, located between the translation start signal and the regiŏn of the structural gene which codes for the fragment of the indicator polypeptide which is essential for its functional activity, which insert causes an out of phase shift in the reading frame of the structural gene or portion thereof and contains a recognition site for a restriction endonuclease.

2. A vector according to claim 1, wherein the recognition site of the oligonucleotide insert is not found elsehwere in the vector.

3. A vector according to claim 1 or claim 2, further comprising a gene which codes for a selectable marker.

4. A vector according to claim 3, wherein the gene codes for a β-lactamase.

5. A vector according to any one of the preceding claims, wherein the indicator polypeptide is β-galactosidase.

6. A vector according to claim 5 which is a recombinant plasmid consisting essentially of, in 5′ to 3′ orientation:

a) a fragment of the plasmid pLG400 containing the gene for ampicillin resistance and the lac z gene or portion of the lac z gene containing at least the region of the gene essential for β-galactosidase activity;

b) a fragment of the plasmid pKB252 located upstream of the lac z gene or portion thereof, containing a lac promoter-operator system and a portion of the phase gene λ CI that contains the ribosomal binding site and translational start signal of the gene λ CI; and

c) an insert located between the translational start signal of the λ CI gene and the region of the lac z gene essential for β-galactosidase activity comprising the oligonucleotide:

5′GATCCCCGGG3′

GGGCCCCTAG.

7. A method for producing a vector containing an exogenous segment of DNA comprising the step of inserting an exogenous segment of DNA at the restriction enzyme site of the insert region of a vector according to any one of claims 1 to 5.

8. A method of cloning and expressing DNA, comprising the steps of:

a) creating polynucleotide fragments of the DNA;

b) inserting the fragments into the insert region of vectors according to claim 1;

c) transforming host cells with the vectors, the host cells having a low level of the indicator polypeptide coded for by the structural gene of the vector;

d) culturing the cells;

e) identifying the cells which exhibit a high level of the indicator polypeptide; and

f) isolating from the identified cells the expressed fused gene product encoded by the vectors.

9. A method according to claim 8, further comprising the steps of:

g) separating from the fused gene product the polypeptide encoded by the inserted DNA; and

h) isolating the polypeptide.

10. A method of making antibodies against polypeptides of a cell or microorganism comprising the steps of:

a) creating polynucleotide fragments of the DNA of the cell or microorganism;

b) inserting the DNA fragments into the insert region of vectors according to claim 1;

c) transforming host cells with the vectors, the host cells having a low level of the indicator polypeptide coded for by the structural gene of the vectors;

d) culturing the cells;

e) identifying the cells which exhibit a high level of the indicator polypeptide;

f) isolating from the identified cells the expressed fused gene product encoded by the vector;

g) raising antibodies against the isolated fusion product; and

h) selecting the antibodies which are reactive with the polypeptide portion of the fused gene product.

11. A method of isolating an open reading frame DNA segment which codes for a polypeptide, comprising the steps of:

a) creating polynucleotide fragments of the DNA;

b) inserting the fragments into the insert region of vectors according to claim 1;

c) transforming host cells with the vectors, the host cells having a low level of the indicator polypeptide coded for by the structural gene of the vectors;

d) culturing the cells;

e) identifying the cells which exhibit a high level of the indicator polypeptide; and

f) isolating from the identified cells the fused gene of the vector containing the open reading frame DNA segment.

12. A method of producing fusion proteins, each containing a polypeptide encoded by a gene segment of an organism and the active fragment of an indicator polypeptide, comprising the steps of:

a) isolating DNA from the organism;

b) generating polynucleotide fragments of the DNA by randomly cleaving the DNA;

c) cleaving vectors according to claim 2 at the unique recognition site with an appropriate restriction endonuclease;

d) forming a fused gene by inserting into the recognition site of the cleaved vectors a polynucleotide fragment;

e) transforming bacterial cells having a low level of indicator activity with the vectors;

f) culturing the cells;

g) selecting the cells which exhibit a high level of indicator activity; and

h) isolating fusion proteins produced by the cells.

**Claims for the Contracting State: AT**

1. A method for producing a vector containing an exogenous segment of DNA comprising the step of inserting an exogenous segment of DNA at the restriction enzyme site of the insert region (c) of a DNA vector consisting essentially of, in 5′ to 3′ orientation:

a) a bacterial promoter, ribosomal binding site and translational start signal;

b) a structural gene coding for an indicator polypeptide (e.g. β-galactosidase), or a portion of the structural gene containing at minimum the region of the gene which codes for a fragment of the indicator polypeptide which is essential for its functional activity; and

c) an oligonucleotide insert, located between the translation start signal and the region of the structural gene which codes for the fragment of the indicator polypeptide which is essential for its functional activity, which insert causes an out of phase shift in the reading frame of the structural gene or portion thereof and contains a recognition site for a restriction endonuclease for example not found elsehwere in the vector.

2. A method according to claim 1, wherein said DNA vector further comprises a gene which codes for a selectable marker, e.g. β-lactamase.

3. A method according to claim 1, wherein said DNA vector is a recombinant plasmid consisting of in 5′ to 3′ orientation:

a) a fragment of the plasmid pLG400 containing the gene for ampicillin resistance and the lac z gene or portion of the lac z gene containing at least the region of the gene essential for β-galactosidase activity;

b) a fragment of the plasmid pKB252 located upstream of the lac z gene or portion thereof, containing a lac promoter-operator system and a portion of the phase gene λ CI that contains the ribosomal binding site and translational start signal of the gene λ CI; and

c) an insert located between the translational start signal of the λ CI gene and the region of the lac z gene essential for β-galactosidase activity comprising the oligonucleotide:

5′GATCCCCGGG3′

GGGCCCCTAG.

4. A method of cloning and expressing DNA, comprising the steps of:

i) creating polynucleotide fragments of the DNA;

ii) inserting the fragments into the insert region (c) of DNA vectors consisting essentially of in 5′ to 3′ orientation:

a) a bacterial promoter, ribosomal binding site and translational start signal;

b) a structural gene coding for an indicator polypeptide, or a portion of the structural gene containing at minimum the region of the gene which codes for a fragment of the indicator polypeptide which is essential for its functional activity; and

c) an oligonucleotide insert, located between the translation start signal and the region of the structural gene which codes for the fragment of the indicator polypeptide which is essential for its functional activity, which insert causes an out of phase shift in the reading frame of the structural gene or portion thereof and contains a recognition site for a restriction endonuclease;

iii) transforming host cells with the vectors, the host cells having a low level of the indicator polypeptide coded for by the structural gene of the vector;

iv) culturing the cells;

v) identifying the cells which exhibit a high level of the indicator polypeptide; and

vi) isolating from the identified cells the expressed fused gene product encoded by the vectors.

5. A method according to claim 4, further comprising the steps of:

vii) separating from the fused gene product the polypeptide encoded by the inserted DNA; and

viii) isolating the polypeptide.

6. A method of making antibodies against polypeptides of a cell or microorganism comprising the steps of:

i) creating polynucleotide fragments of the DNA of the cell or microorganism;

ii) inserting the DNA fragments into the insert region (c) of DNA vectors consisting essentially of, in 5′ to 3′ orientation:

a) A bacterial promoter, ribosomal binding site and translational start signal;

b) a structural gene coding for an indicator polypeptide, or a portion of the structural gene containing at minimum the region of the gene which codes for a fragment of the indicator polypeptide which is essential for its functional activity; and

c) an oligonucleotide insert, located between the translation start signal and the region of the structural gene which codes for the fragment of the indicator polypeptide which is essential for its functional activity, which insert causes an out of phase shift in the reading frame of the structural gene or portion thereof and contains a recognition site for a restriction endonuclease;

iii) transforming host cells with the vectors, the host cells having a low level of the indicator polypeptide coded for by the structural gene of the vectors;

iv) culturing the cells;

v) identifying the cells which exhibit a high level of the indicator polypeptide;

vi) isolating from the identified cells expressed fused gene product encoded by the vector;

vii) raising antibodies against the isolated fusion product; and

viii) selecting the antibodies which are reactive with the polypeptide portion of the fused gene product.

7. A method of isolating an open reading frame DNA segment which codes for a polypeptide, comprising the steps of:

i) creating polynucleotide fragments of the DNA;

ii) inserting the fragments into the insert region (c) of vectors consisting essentially of, in 5′ to 3′ orientation:

a) a bacterial promoter, ribosomal binding site and translational start signal;

b) a structural gene coding for an indicator polypeptide, or a portion of the structural gene containing at minimum the region of the gene which codes for a fragment of the indicator polypeptide which is essential for its functional activity; and

c) an oligonucleotide insert, located between the translation start signal and the region of the structural gene which codes for the fragment of the indicator polypeptide which is essential for its functional activity, which insert causes an out of phase shift in the reading frame of the structural gene or portion thereof and contains a recognition site for a restriction endonuclease;

iii) transforming host cells with the vectors, the host cells having a low level of the indicator polypeptide coded for by the structural gene of the vectors;

iv) culturing the cells;

v) identifying the cells which exhibit a high level of the indicator polypeptide; and

vi) isolating from the identified cells the fused gene of the vector containing the open reading frame DNA segment.

8. A method of producing fusion proteins, each containing a polypeptide encoded by a gene segment of an organism and the active fragment of an indicator polypeptide, comprising the steps of:

i) isolating DNA from the organism;

ii) generating polynucleotide fragments of the DNA by randomly cleaving the DNA;

iii) cleaving with an appropriate restriction endonuclease at the unique recognition site of the insert region (c) of DNA vectors consisting essentially of, in 5′ to 3′ orientation.

a) a bacterial promoter, ribosomal binding site and translational start signal;

b) a structural gene coding for an indicator polypeptide, or a portion of the structural gene containing at minimum the region of the gene which codes for a fragment of the indicator polypeptide which is essential for its functional activity; and

c) an oligonucleotide insert, located between the translation start signal and the region of the structural

gene which codes for the fragment of the indicator polypeptide which is essential for its functional activity, which insert causes an out of phase shift in the reading frame of the structural gene or portion thereof and contains a recognition site for a restriction endonuclease not found elsewhere in the vector;

iv) forming a fused gene by inserting into the recognition site of the cleaved vectors a polynucleotide fragment;

v) transforming bacterial cells having a low level of indicator activity with the vectors;

vi) culturing the cells;

vii) selecting the cells which exhibit a high level of indicator activity; and

viii) isolating fusion proteins produced by the cells.

**Patentansprüche für die Vertragsstaaten: BE CH DE FR GB LI LU NL SE**

1. DNS-Vektor, im wesentlichen, in 5'- nach 3'-Richtung, bestehend aus:

a) einem bakteriellen Promoter, einem ribosomalen Bindungsort und einem Translationsstartsignal;

b) einem ein Indikatorpolypeptid codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält.

2. Vektor nach Anspruch 1, bei dem der Erkennungsort des Oligonukleotideinsatzes nicht irgendwoanders im Vektor anzutreffen ist.

3. Vektor nach Anspruch 1 oder Anspruch 2, weiterhin mit einem Gen, das einen auswählbaren Marker codiert.

4. Vektor nach Anspruch 3, bei dem das Gen eine β-Lactamase codiert.

5. Vektor nach einem der vorhergehenden Ansprüche, bei dem das Indikatorpolypeptid aus β-Galactosidase besteht.

6. Vektor nach Anspruch 5, als rekombinantes Plasmid im wesentlichen, in 5'- nach 3'-Richtung, bestehend aus:

a) einem Bruchstück des Plasmids pLG400 mit dem Gen für Ampicillinresistenz und dem lac-z-Gen oder einem Teil des lac-z-Gens, der zumindest die für β-Galactosidaseaktivität wesentliche Genregion enthält;

b) einem stromaufwärts des lac-z-Gens oder eines Teils desselben angeordneten Bruchstück des Plasmids pKB252 mit einem lac-Promoter-Operator-System und einem Teil des Phagengens λ CI, das den ribosomalen Bindungsort und das Translationsstartsignal des Gens λ CI enthält; und

c) einem zwischen der Translationsstartsignal des Gens λ CI und der für β-Galactosidaseaktivität wesentlichen Region des lac-z-Gens angeordneten Einsatz mit dem Oligonukleotid:

5'GATCCCCGGG3'

GGGCCCCTAG.

7. Verfahren zur Herstellung eines ein exogenes DNS-Segment enthaltenden Vektors, mit dem Verfahrensschritt, daß ein exogenes DNS-Segment an dem Restriktionsenzymort der Einsatzregion eines Vektors nach einem der Ansprüche 1 bis 5 eingesetzt wird.

8. Verfahren zum Klonieren und Ausdrücken von DNS, bestehend aus folgenden Schritten:

a) es werden Polynukleotid-Bruchstücke der DNS erzeugt;

b) die Bruchstücke werden in die Einsatzregion von Vektoren nach Anspruch 1 eingesetzt;

c) Wirtszellen werden mit den Vektoren transformiert, wobei die Wirtszellen ein niedriges Niveau von durch das Strukturgen des Vektors codiertem Indikatorpolypeptid besitzen;

d) die Zellen werden zur Vermehrung gebracht;

e) diejenigen Zellen, die ein hohes Niveau an Indikatorpolypeptid, zeigen, werden identifiziert; und

f) von den identifizierten Zellen wird das von den Vektoren codierte ausgedrückte Fusionsgenprodukt abgesondert.

9. Verfahren nach Anspruch 8, mit dem weiteren Verfahrensschritt, daß

g) das von der eingesetzten DNS codierte Polypeptid von dem Fusionsgenprodukt getrennt und

h) das Polypeptid abgesondert wird.

10. Verfahren zur Herstellung von Antikörpern gegen Polypeptide einer Zelle oder eines Mikroorganismus, bestehend aus folgenden Verfahrensschritten:

a) es werden Polynukleotid-Bruchstücke der DNS der Zellen oder des Mikroorganismus erzeugt;

b) die DNS-Bruchstücke werden in die Einsatzregion der Vektoren nach Anspruch 1 eingesetzt;

c) Wirtszellen werden mit den Vektoren transformiert, wobei die Wirtszellen ein niedriges Niveau an durch das Strukturgen der Vektoren codiertem Indikatorpolypeptid besitzen;

d) die Zellen werden zur Vermehrung gebracht;

e) diejenigen Zellen, die ein hohes Niveau an Indikatorpolypeptid zeigen, werden identifiziert;

f) von den identifizierten Zellen wird das von dem Vektor codierte ausgedrückte Fusionsgenprodukt abgesondert;

g) es werden Antikörper gegen das isolierte Fusionsprodukt aufgezogen; und

h) es werden die Antikörper ausgewählt, die mit dem Polypeptidanteil des Fusionsgenprodukts reaktionsfähig sind.

11. Verfahren zur Absonderung eines DNS-Segments mit ofenem Leseraster, welches ein Polypeptid codiert, bestehend aus folgenden Schritten:

a) es werden Polynukleotid-Bruchstücke der DNS erzeugt;

b) die Bruchstücke werden in die Einsatzregion der Vektoren nach Anspruch 1 eingesetzt;

c) Wirtszellen werden mit den Vektoren transformiert, wobei die Wirtszellen ein niedriges Niveau an durch das Strukturgen der Vektoren codiertem Indikatorpolypeptid besitzen;

d) die Zellen werden zur Vermehrung gebracht;

e) es werden die Zellen identifiziert, die ein hohes Niveau an Indikatorpolypeptid zeigen; und

f) von den identifizierten Zellen wird das Fusionsgen des Vektors abgesondert, das das DNS-Segment mit offenem Leseraster enthält.

12. Verfahren zur Herstellung von Fusionsproteinen, die jeweils ein Polypeptid enthalten, das durch ein Gensegment eines Organismus und das aktive Bruchstück eines Indikatorpolypeptids codiert ist, bestehend aus folgenden Schritten:

a) von dem Organismus wird DNS abgesondert;

b) es werden Polynukletid-Bruchstücke der DNS durch ungerichtetes Aufspalten der DNS erzeugt;

c) es werden Vektoren nach Anspruch 2 an dem einzigen Erkennungsort mit einer angemessenen Restriktionsendonuklease aufgespalten;

d) es wird ein Fusionsgen gebildet, indem in den Erkennungsort der aufgespaltenen Vektoren ein Polynukleotid-Bruchstück eingesetzt wird;

e) bakterielle Zellen mit einem niedrigen Niveau an Indikatioraktivität werden mit den Vektoren transformiert;

f) die Zellen werden zur Vermehrung gebracht;

g) die Zellen, die ein hohes Niveau an Indikatoraktivität zeigen, werden ausgewählt; und

h) die von den Zellen produzierten Fusionsproteine werden abgesondert.

**Patentansprüche für den Vertragsstaat: AT**

1. Verfahren zur Herstellung eines ein exogenes DNS-Segment enthaltenden Vektors, mit dem Schritt des Einsetzens eines exogenen DNS-Segments am Restriktionsenzymort der Einsatzregion (c) eines DNS-Vektors, im wesentlichen, in 5'- nach 3'-Richtung, bestehend aus:

a) einem bakteriellen Promoter, einem ribosomalen Bindungsort und einem Translationsstartsignal;

b) einem ein Indikatorpolypeptid (z.B. β-Galactosidase) codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält, der zum Beispiel nicht irgendwoanders im Vektor anzutreffen ist.

2. Verfahren nach Anspruch 1, bei dem der DNS-Vektor ferner ein Gen umfaßt, das einen auswählbaren Marker, z.B. β-Laktamase codiert.

3. Verfahren nach Anspruch 1, bei dem der DNS-Vektor ein rekombinantes Plasmid ist, im wesentlichen, in 5'- nach 3'-Richtung, bestehend aus:

a) einem Bruchstück des Plasmids pLG400 mit dem Gen für Ampicillinresistenz und dem lac-z-Gen oder einem Teil des lac-z-Gens, der zumindest die für β-Galactosidaseaktivität wesentliche Genregion enthält;

b) einem stromaufwärts des lac-z-Gens oder eines Teils desselben angeordneten Bruchstück des Plasmids pKB252 mit einem lac-Promoter-Operator-System und einem Teil des Phagengens λ CI, das den ribosomalen Bindungsort und das Translationsstartsignal des Gens λ CI enthält; und

c) einem zwischen der Translationsstartsignal des Gens λ CI und der für β-Galactosidaseaktivität wesentlichen Region des lac-z-Gens angeordneten Einsatz mit dem Oligonukleotid:

5'GATCCCCGGG3'

GGGCCCCTAG.

4. Verfahren zum Klonieren und Ausdrücken von DNS, bestehend aus folgenden Schritten:

i) es werden Polynukleotid-Bruchstücke der DNS erzeugt;

ii) die Bruchstücke werden in die Einsatzregion (c) von DNS-Vektoren eingesetzt, die, im wesentlichen, in 5'- nach 3'-Richtung, bestehen aus:

a) einem bakteriellen Promoter, einem ribosomalen Bindungsort und einem Translationsstartsignal;

b) einem ein Indikatorpolypeptid codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält.

iii) Wirtszellen werden mit den Vektoren transformiert, wobei die Wirtszellen ein niedriges Niveau von durch das Strukturgen des Vektors codiertem Indikatorpolypeptid besitzen;

iv) die Zellen werden zur Vermehrung gebracht;

v) diejenigen Zellen, die ein hohes Niveau an Indikatorpolypeptid zeigen, werden identifiziert; und

vi) von den identifizierten Zellen wird das von den Vektoren codierte ausgedrückte Fusionsgenprodukt abgesondert.

5. Verfahren nach Anspruch 4, mit dem weiteren Verfahrensschritten, daß

vii) das von der eingesetzten DNS codierte Polypeptid von dem Fusionsgenprodukt getrennt und

viii) das Polypeptid abgesondert wird.

6. Verfahren zur Herstellung von Antikörpern gegen Polypeptide einer Zelle oder eines Mikroorganismus, bestehend aus folgenden Verfahrensschritten:

i) es werden Polynukleotid-Bruchstücke der DNS der Zelle oder des Mikroorganismus erzeugt;

ii) die DNS-Bruchstücke werden in die Einsatzregion (c) der DNS-Vektoren eingesetzt, die im wesentlichen, in 5'- nach 3'-Richtung, bestehen aus

a) einem bakteriellen Promotor, einem ribosomalen Bindungsort und einem Translationsstartsignal;

b) einem ein Indikatorpolypeptid codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält.

iii) Wirtszellen werden mit den Vektoren transformiert, wobei die Wirtszellen ein niedriges Niveau an durch das Strukturgen der Vektoren codiertem Indikatorpolypeptid besitzen;

iv) die Zellen werden zur Vermehrung gebracht;

v) diejenigen Zellen, die ein hohes Niveau an Indikatorpolypeptid zeigen, werden identifiziert;

vi) von den identifizierten Zellen wird das von dem Vektor codierte ausgedrückte Fusionsgenprodukt abgesondert;

vii) es werden Antikörper gegen das isolierte Fusionsprodukt aufgezogen; und

viii) es werden die Antikörper ausgewählt, die mit dem Polypeptidanteil des Fusionsgenprodukts reaktionsfähig sind.

7. Verfahren zur Absonderung eines DNS-Segments mit offenem Leseraster, welches ein Polypeptid codiert, bestehend aus folgenden Schritten:

i) es werden Polynukleotid-Bruchstücke der DNS erzeugt;

ii) die Bruchstücke werden in die Einsatzregion (c) von Vektoren eingesetzt, die im wesentlichen, in 5'-nach 3'-Richtung, bestehen aus:

a) einem bakteriellen Promotor, einem ribosomalen Bindungsort und einem Translationsstartsignal;

b) einem ein Indikatorpolypeptid codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält.

iii) Wirtszellen werden mit den Vektoren transformiert, wobei die Wirtszellen ein niedriges Niveau an durch das Strukturgen der Vektoren codiertem Indikatorpolypeptid besitzen;

iv) die Zellen werden zur Vermehrung gebracht;

v) es werden die Zellen identifiziert, die ein hohes Niveau an Indikatorpolypeptid zeigen; und

vi) von den identifizierten Zellen wird das Fusionsgen des Vektors abgesondert, das das DNS-Segment mit offenem Leseraster enthält.

8. Verfahren zur Herstellung von Fusionsproteinen, die jeweils ein Polypeptid enthalten, das durch ein

Gensegment eines Organismus und das aktive Bruchstück eines Indikatorpolypeptids codiert ist, bestehend aus folgenden Schritten:

i) von dem Organismus wird DNS abgesondert;

ii) es werden Polynukleotid-Bruchstücke der DNS durch ungerichtetes Aufspalten der DNS erzeugt;

iii) es werden mit einer angemessenen Restriktionsendonuklease an dem einzigen Erkennungsort der Einsatzregion (c) DNS-Vektoren aufgespalten, die im wesentlichen, in 5'- nach 3'-Richtung, bestehen aus:

a) einem bakteriellen Promotor, einem ribosomalen Bindungsort und einem Translationsstartsignal;

b) einem ein Indikatorpolypeptid codierenden Strukturgen oder einem Teil des Strukturgens, der zumindest die Genregion enthält, die ein Bruchstück des Indikatorpolypeptids codiert, das für seine Funktionsaktivität wesentlich ist; und

c) einem Oligonukleotideinsatz, der zwischen dem Translationsstartsignal und der Region des Strukturgens angeordnet ist, die das Bruchstück des Indikatorpolypeptids codiert, welches wesentlich für seine Funktionsaktivität ist, wobei der Einsatz eine Außerphasenverschiebung im Leseraster des Strukturgens oder eines Teils desselben hervorruft und einen Erkennungsort für eine Restriktionsendonuklease enthält.

iv) es wird ein Fusionsgen gebildet, indem in den Erkennungsort der aufgespaltenen Vektoren ein Polynukleotidbruchstück eingesetzt wird;

v) bakterielle Zellen mit einem niedrigen Niveau an Indikatoraktivität werden mit den Vektoren transformiert;

vi) die Zellen werden zur Vermehrung gebracht;

vii) die Zellen, die ein hohes Niveau an Indikatoraktivität zeigen, werden ausgewählt; und

viii) die von den Zellen produzierten Fusionsproteine werden abgesondert.

**Revendications pour les Etats Contractants: BE CH DE FR GB LI LU NL SE**

1. Vecteur d'ADN, se composant essentiellement, dans le sens 5' → 3', de:

a) un promoteur bacterien, site de fixation ribosomiale et signal de démarrage de translation;

b) un gène de structure codant pour un polypeptide indicateur ou une partie du gène de structure contenant au minimum la région du gène qui code pour un fragment du polypeptide indicateur qui est essentiel pour son activité fonctionnelle; et

c) un insert obligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction.

2. Vecteur selon la revendication 1, caractérisé en ce que le site de reconnaissance de l'insert oligonucléotidique ne se trouve pas ailleurs dans le vecteur.

3. Vecteur selon la revendication 1 ou 2, caractérisé en ce qu'il comprend en outre un gène qui code pour un marqueur sélectionnable.

4. Vecteur selon la revendication 3, caractérisé en ce que le gène code pour une β-lactamase.

5. Vecteur selon l'une quelconque des revendications 1 à 4, caractérisé en ce que le polypeptide indicateur est une β-galactosidase.

6. Vecteur selon la revendication 5, qui est un plasmide recombinant se composant essentiellement, dans le sens 5' → 3', de:

a) un fragment du plasmide pLG400 contenant le gène pour la résistance à l'ampicilline et le gène lac z ou une partie du gène lac z contenant au moins la région du gène essentielle pour l'activité de β-galactosidase;

b) un fragment du plasmide pKB252 situé en amont du gène lac z ou de la partie de celui-ci, contenant un système lac-promoteur-opérateur et une partie du gène du phage λCI qui contient le site de fixation ribosomiale et le signal de démarrage de translation du gène λCI; et

c) un insert, situé entre le signal de démarrage de translation du gène λCI et la région du gène lac z essentielle pour l'activité de β-galactosidase, constitué par l'oligonucléotide

5'GATCCCCGGG3'

GGGCCCCTAG.

7. Procédé de fabrication d'un vectuer contenant un segment exogène d'ADN, comprenant l'opération consistant à insérer un segment exogène d'ADN au niveau du site d'enzyme de restriction de la région d'insert d'un vecteur selon l'une quelconque des revendications 1 à 5.

8. Procédé de clonage et d'expression d'ADN, comprenant les operations successives consistant à:

a) créer des fragments polynucleotidiques de l'ADN;

b) insérer les fragments dans la région de l'insert de vecteur selon la revendication 1;

c) transformer des cellules-hôtes avec les vecteurs, les cellules-hôtes ayant une faible teneur en le polypeptide indicateur pour lequel code le gène de structure du vecteur;

d) cultiver les cellules;

e) identifier les cellules qui presentent une forte teneur en le polypeptide indicateur; et

f) isoler des cellules identifiees le produit gène fondu exprimé qui est codé par les vecteurs.

9. Procédé selon la revendication 8, comprenant en outre les opérations consistant à:

g) séparer du produit gène fondu le polypeptide codé par l'ADN inséré; et

h) isoler le polypeptide.

10. Procédé de fabrication d'anticorps à l'égard de polypeptides d'une cellule ou d'un micro-organisme, comprenant les opération successives consistant à:

a) créer des fragments polynucleotidiques de l'ADN de la cellule ou du micro-organisme;

b) insérer les fragments d'ADN dans la région d'insert de vecteurs selon la revendication 1;

c) transformer des cellules-hôtes avec les vecteurs, les cellules-hôtes ayant une faible teneur en le polypeptide indicateur pour lequel code le gène de structure des vecteurs;

d) cultiver les cellules;

e) identifier les cellules qui présentent une forte teneur en le polypeptide indicateur;

f) isoler des cellules identifiées le produit gène fondu exprimé qui est codé par les vecteurs;

g) produire des anticorps à l'égard du produit de fusion isolé; et

h) sélectionner les anticorps qui sont réactifs avec la partie polypeptitique du produit gène fondu.

11. Procédé d'isolement d'un segment d'ADN à cadre de lécture ouvert qui code pour un polypeptide, comprenant les opérations successives consistant à:

a) créer des fragments polynucléotidiques de l'ADN;

b) insérer les fragments dans la région d'insert de vecteurs selon la revendication 1;

c) transformer des cellules-hôtes avec les vecteurs, les cellules-hôtes ayant une faible teneur en le polypeptide indicateur pour lequel code le gène de structure des vecteurs;

d) cultiver les cellules;

e) identifier les cellules qui présentent une forte teneur en le polypeptide indicateur; et

f) isoler des cellules identifiées le gène fondu du vecteur contenant le segment d'ADN à cadre de lecture ouvert.

12. Procédé de fabrication de protéines de fusion, contenant chacune un polypeptide codé par un segment de gène d'un organisme et le fragment actif d'un polypeptide indicateur, comprenant les opérations successives consistant à

a) isoler l'ADN de l'organisme;

b) produire des fragments polynucléotidiques de l'ADN par coupure au hasard de l'ADN;

c) couper des vecteurs selon la revendication 2 au niveau du site de reconnaissance unique, au moyen d'une endonucléase de restriction appropriée;

d) former un gène fondu en insérant un fragment polynucléotidique dans le site de reconnaissance des vecteurs coupés;

e) transformer avec les vecteurs des cellules bactériennes ayant un bas niveau d'activite d'indicateur;

f) cultiver les cellules;

g) sélectionner les cellules qui présentent un niveau élevé d'activité d'indicateur; et

h) isoler les proteines de fusion produites par les cellules.

**Revendications pour l'Etat Contractant: AT**

1. Procédé de fabrication d'un vecteur contenant un segment exogène d'ADN, comprenant l'opération consistant à insérer un segment exogène d'ADN au niveau du site d'enzyme de restriction de la région d'insert (c) d'un vecteur d'ADN se composant essentiellement, dans le sens 5' → 3', de:

a) un promoteur bacterien, site de fixation ribosomiale et signal de démarrage de translation;

b) un gène de structure codant pour un polypeptide indicateur (par exemple une β-galactosidase) ou une partie du gène de structure contenant au minimum la région du gène qui code pour un fragment du polypeptide indicateur qui est essentiel pour son activité fonctionnelle; et

c) un insert oligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction par exemple qui ne se trouve pas ailleurs dans le vecteur.

2. Procédé selon la revendication 1, caractérisé en ce que ledit vecteur d'ADN comprend en outre un gène qui code pour un marqueur selectionnable, par exemple une β-lactamase.

3. Procédé selon la revendication 1, caractérisé en ce que ledit vecteur d'ADN est un plasmide recombinant se composant , dans le sens 5' → 3', de:

a) un fragment du plasmide pLG400 contenant le gène pour la résistance à l'ampicilline et le gène lac z ou une partie du gène lac z contenant au moins la région du gène essentielle pour l'activité de β-galactosidase;

b) un fragment du plasmide pKB252 situé en amont du gène lac z ou de la partie de celui-ci, contenant un système lac-promoteur-opérateur et une partie du gène du phage λCI qui contient le site de fixation ribosomiale et le signal de démarrage de translation du gène λCI; et

c) un insert, situé entre le signal de démarrage de translation du gène λCI et la région du gène lac z essentielle pour l'activité de β-galactosidase, constitué par l'oligonucléotide

5'GATCCCCGGG3'
GGGCCCCTAG.

4. Procédé de clonage et d'expression d'ADN, comprenant les operations successives consistant à:

i) créer des fragments polynucleotidiques de l'ADN;

ii) insérer les fragments dans la région de l'insert (c) de vecteurs d'ADN se composant essentiellement, dans la sens 5' → 3', de:

a) un promoteur bacterien, site de fixation ribosomiale et signal de démarrage de translation;

b) un gène de structure codant pour un polypeptide indicateur ou une partie gène de structure contenant au minimum la région du gène qui code pour un fragment du polypeptide indicateur qui est essentiel pour son activité fonctionnelle; et

c) un insert oligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction;

iii) transformer des cellules-hôtes avec les vecteurs, les cellules-hôtes ayant une faible teneur en le polypeptide indicateur pour lequel code le gène de structure du vecteur;

iv) cultiver les cellules;

v) identifier les cellules qui presentent une forte teneur en le polypeptide indicateur; et

f) isoler des cellules identifiees le produit de gène fondu exprimé qui est codé par les vecteurs.

5. Procédé selon la revendication 4, comprenant en outre les opérations consistant à:

vii) séparer du produit gène fondu le polypeptide codé par l'ADN inséré; et

viii) isoler le polypeptide.

6. Procédé de fabrication d'anticorps à l'égard de polypeptides d'une cellule ou d'un micro-organisme, comprenant les opération successives consistant à:

i) créer des fragments polynucleotidiques de l'ADN de la cellule ou du micro-organisme;

ii) insérer les fragments d'ADN dans la région d'insert (c) de vecteurs d'ADN se composant essentiellement, dans le sens 5' → 3', de:

a) un promoteur bacterien, site de fixation ribosomiale et signal de démarrage de translation;

b) un gène de structure codant pour un polypeptide indicateur ou une partie du gène de structure contenant au minimum la région du gène qui code pour un fragment du polypeptide indicateur qui est essentiel pour son activité fonctionnelle; et

c) un insert oligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction;

iii) transformer des cellules-hôtes avec les vecteurs, les cellules-hôtes ayant une faible teneur en le polypeptide indicateur pour lequel code le gène de structure des vecteurs;

iv) cultiver les cellules;

v) identifier les cellules qui présentent une forte teneur en le polypeptide indicateur;

vi) isoler des cellules identifiées le produit gène fondu exprimé qui est codé par les vecteurs;

vii) produire des anticorps à l'égard du produit de fusion isolé; et

viii) sélectionner les anticorps qui sont réactifs avec la partie polypeptitique du produit gène fondu.

7. Procédé d'isolement d'un segment d'ADN à cadre de lécture ouvert qui code pour un polypeptide, comprenant les opérations successives consistant à:

i) créer des fragments polynucléotidiques de l'ADN;

ii) insérer les fragments dans la région d'insert (c) de vecteurs se composant essentiellement, dans le sens 5' → 3', de:

a) un promoteur bacterien, site de fixation ribosomiale et signal de démarrage de translation;

b) un gène de structure codant pour un polypeptide indicateur ou une partie gène de structure contenant au minimum la région du gène qui code pour un fragment du polypeptide indicateur qui est essentiel pour son activité fonctionnelle; et

c) un insert oligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction;

iii) transformer des cellules-hôtes avec les vecteurs, les cellules-hôtes ayant une faible teneur en le polypeptide indicateur pour lequel code le gène de structure des vecteurs;

iv) cultiver les cellules;

v) identifier les cellules qui présentent une forte teneur en le polypeptide indicateur; et

f) isoler des cellules identifiées le gène fondu du vecteur contenant le segment d'ADN à cadre de lecture ouvert.

8. Procédé de fabrication de protéines de fusion, contenant chacune un polypeptide codé par un segment de gène d'un organisme et le fragment actif d'un polypeptide indicateur, comprenant les opérations successives consistant à

i) isoler l'ADN de l'organisme;

ii) produire des fragments polynucléotidiques de l'ADN par coupure au hasard de l'ADN;

iii) couper, au moyen d'une endonucléase de restriction appropriee, au niveau du site de reconnaissance unique, des vecteurs d'ADN se composant essentiellement, dans le sens 5' → 3', de:

a) un promoteur bacterien, site de fixation ribosomiale et signal de démarrage de translation;

b) un gène de structure codant pour un polypeptide indicateur ou une partie du gène de structure contenant au minimum la région du gène qui code pour un fragment du polypeptide indicateur qui est essentiel pour son activité fonctionnelle; et

c) un insert oligonucléotidique, situé entre le signal de démarrage de translation et la région du gène de structure qui code pour le fragment du polypeptide indicateur qui est essentiel pour son activité fonctionnelle, insert qui cause un décalage dephasé dans le cadre de lecture du gène de structure ou de la partie de celui-ci et contient un site de reconnaissance pour une endonucléase de restriction;

iv) former un gène fondu en insérant un fragment polynucléotidique dans le site de reconnaissance des vecteurs coupés;

v) transformer avec les vecteurs des cellules bactériennes ayant un bas niveau d'activite d'indicateur;

vi) cultiver les cellules;

vii) sélectionner les cellules qui présentent un niveau élevé d'activité d'indicateur; et

viii) isoler les proteines de fusion produites par les cellules.

*Fig. 1*

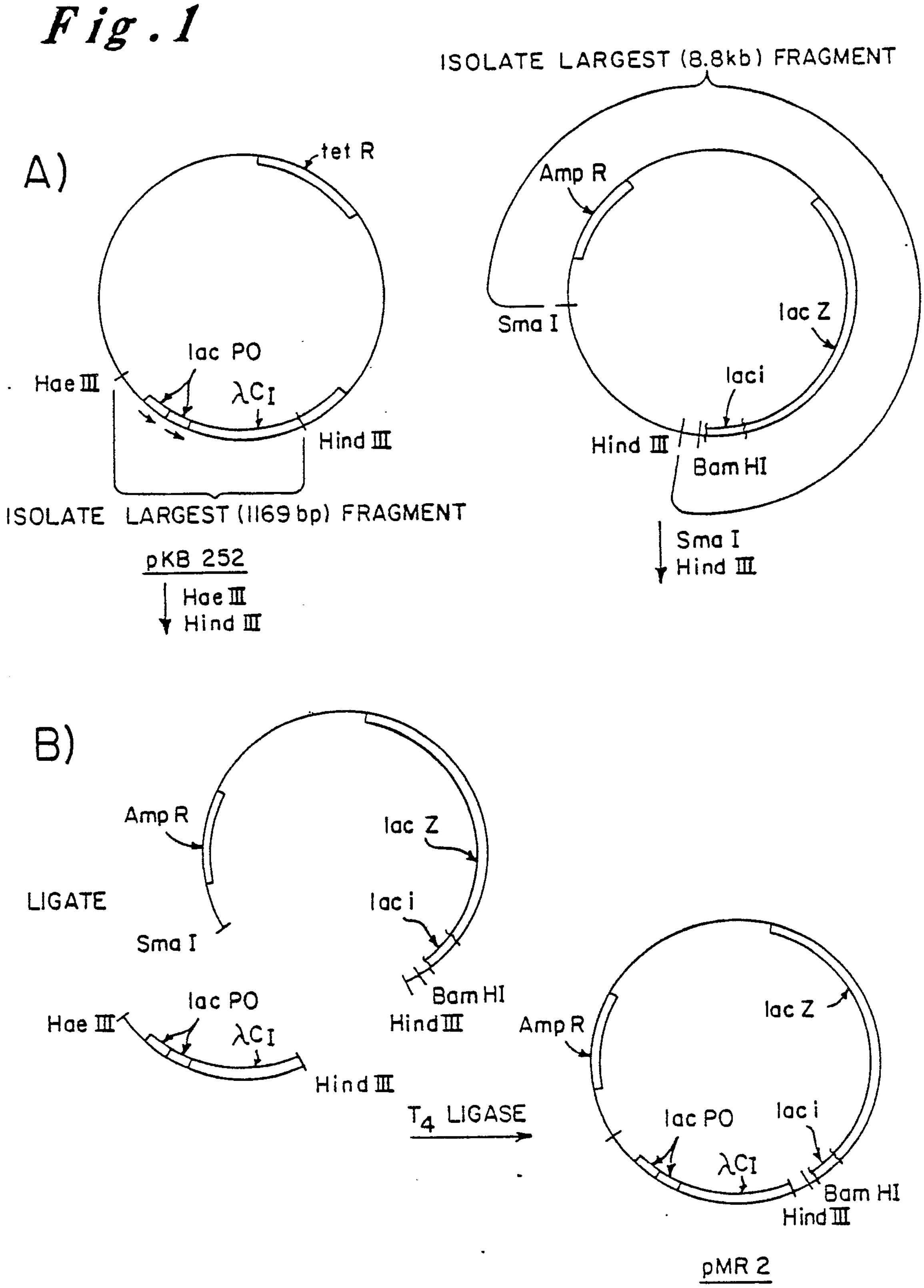
ISOLATE LARGEST (8.8kb) FRAGMENT
A)
tet R
Amp R
Sma I
lac Z
lac PO
lac i
Hae III
λCI
Hind III
Bam HI
ISOLATE LARGEST (1169 bp) FRAGMENT
pKB 252
Hae III
Hind III
Sma I
Hind III
B)
Amp R
lac Z
LIGATE
lac i
Sma I
Bam HI
lac PO
Hae III
λCI
Hind III
Amp R
lac Z
Hind III
T4 LIGASE
lac PO
lac i
λCI
Bam HI
Hind III
pMR 2

*Fig.2*

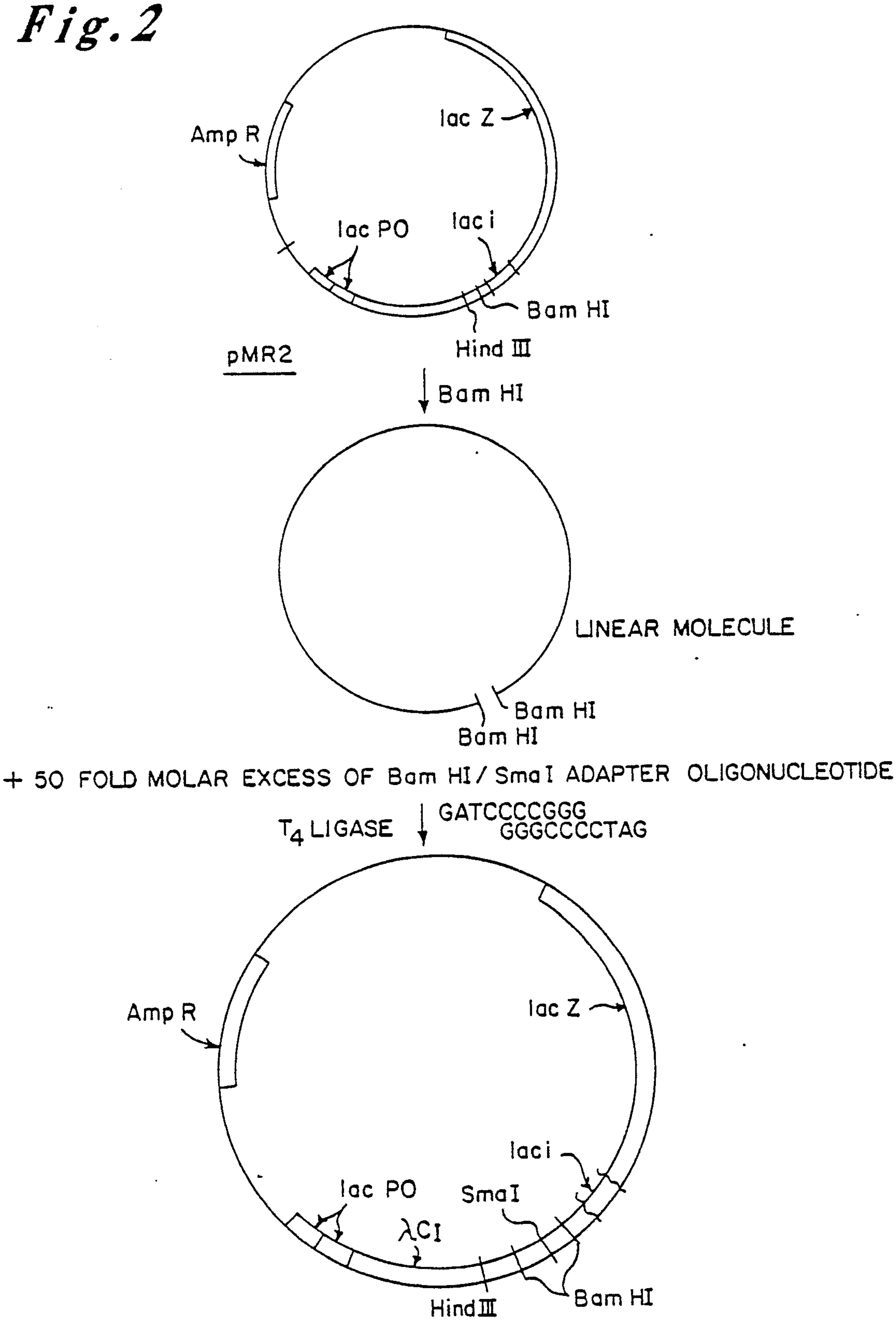
Amp R
lac Z
lac PO
lac i
Bam HI
Hind III
pMR2
Bam HI
LINEAR MOLECULE
Bam HI
Bam HI
+ 50 FOLD MOLAR EXCESS OF Bam HI / Sma I ADAPTER OLIGONUCLEOTIDE
$T_4$ LIGASE
GATCCCCGGG
GGGCCCCTAG
Amp R
lac Z
lac i
lac PO
Sma I
λCI
Hind III
Bam HI